# EUROPÄISCHE PATENTSCHRIFT

(11) **EP 0 991 656 B1**
(45) Veröffentlichungstag und Bekanntmachung des Hinweises auf die Patenterteilung: **04.09.2002**
(21) Anmeldenummer: 98936343.7
(22) Anmeldetag: 15.06.1998
(51) Int. Cl.: C07F 9/54, C07C 37/66, C07C 39/02

(54) **VERFAHREN ZUR HERSTELLUNG VON PHOSPHONIUMPHENOLATEN**
METHOD FOR PRODUCING PHOSPHONIUM PHENOLATES
PROCEDE DE PREPARATION DE PHENOLATES DE PHOSPHONIUM

(30) Priorität: 27.06.1997 DE 19727351
(43) Veröffentlichungstag der Anmeldung: 12.04.2000
(73) Patentinhaber: Bayer Aktiengesellschaft, 51368 Leverkusen (DE)
(72) Erfinder: KÖNIG, Annett, D-47800 Krefeld (DE); BUNZEL, Lothar, D-47906 Kempen (DE); HUCKS, Uwe, D-46519 Alpen (DE)
(86) Internationale Anmeldenummer: EP9803590
(87) Internationale Veröffentlichungsnummer: WO99000395

(56) Entgegenhaltungen:
- EP-A- 0 826 693
- US-A- 4 340 761

## Beschreibung

Gegenstand der deutschen Patentanmeldung P 196 35 656.3 ist ein Verfahren zur Herstellung von Phosphoniumphenolaten, das dadurch gekennzeichnet ist, daß man Phosphoniumhalogenide mit Phenolen in einem Gemisch aus Wasser, wäßriger Alkalilauge und inertem Lösungsmittel zwischen 2 Minuten und 4 Stunden bei Temperaturen von 0°C bis 40°C und bei Drücken von 1 bar bis 20 bar im Molverhältnis von 0,5 mol bis 2 mol Phenol, vorzugsweise von 0,7 bis 1,3 mol Phenol, pro Mol Phosphoniumhalogenid, umsetzt.

Als inerte Lösungsmittel sind darin CH₂Cl₂ und Cl-C₆H₅ genannt.

Auf der Seite 4 der deutschen Patentanmeldung P 196 35 656.3 wird außerdem festgestellt, daß Phosphoniumsalze durch Basen in das entsprechende Phosphinoxid überführt werden. In Beispiel A dieser Anmeldung wird dies belegt, wobei bei einem pH-Wert von ca. 14 gearbeitet wird. (Siehe auch Vergleichsbeispiel dieser Anmeldung sowie deutsche Anmeldung P 19723524.7, die mit innerer Priorität der P 19635656.3 am 05.06.1997 eingereicht wurde).

Durch Weiterentwicklung dieses Verfahrens wurde nun gefunden, daß anstelle von inerten Lösungsmitteln Alkohole eingesetzt werden, die in Wasser nur bis maximal 15 Gew.-% löslich sind.

Es wurde außerdem überraschend gefunden, daß die Bildung von Phosphinoxiden dann unterdrückt wird, wenn man in alkalischer Lösung auch ohne Alkohol bei einem pH-Wert zwischen 9,5 und 11, bevorzugt zwischen 9,5 und 10,5 und besonders bevorzugt zwischen 10,0 und 10,5 arbeitet.

Gegenstand der vorliegenden Erfindung ist somit ein Verfahren zur Herstellung von Phosphoniumphenolaten aus Phosphoniumhalogeniden und Phenolen in wäßrig-alkalischer Lösung bei Temperaturen von 0 bis 55°C, vorzugsweise von 15 bis 50°C, das dadurch gekennzeichnet ist, daß man die Umsetzung im Molverhältnis Phenol zu Phosphoniumhalogenid zwischen 2:1 und 10:1, vorzugsweise zwischen 4,5:1 und 6:1 und insbesondere 5:1, bei pH-Werten von 9,5 bis 11, vorzugsweise von 9,5 bis 10,5 und insbesondere von 10 bis 10,5 und in Gegenwart von Alkoholen in Mengen von 50 Gew.-% bis 200 Gew.-%, vorzugsweise von 66 Gew.-% bis 125 Gew.-%, bezogen auf die Masse der wäßrigen Phase durchführt, wobei die Alkohole eine Löslichkeit in reinem Wasser von maximal 15 Gew.-% haben.

Mit dem erfindungsgemäßen Verfahren gelingt es Phosphoniumphenolate in hohen Ausbeuten herzustellen.

Phosphoniumphenolate eignen sich besonders als Katalysatoren zur Veresterung und zur Umesterung, insbesondere zur Herstellung von Polycarbonaten nach dem Schmelzumesterungsverfahren (siehe US-A 3 442 854).

Die Löslichkeit der Alkohole in Wasser ist literaturbekannt.

Erfindungsgemäß geeignete Alkohole sind aliphatische der Formel CₙH₂ₙ₊₁-OH, worin n eine ganze Zahl von 3 bis 10 einschließlich ist.

Erfindungsgemäß geeignete Alkohole sind auch cycloaliphatische der Formel CₙH₂ₙ₋₁-OH, worin n eine ganze Zahl von 5 bis 10 einschließlich ist.

Bevorzugte aliphatische Alkohole sind (Iso)Butanole, Pentanole und Hexanole, insbesondere Isobutanol.

Bevorzugte cycloaliphatische Alkohole sind Cyclopentanol, Cycloheptanol und Cyclooctanol, besonders bevorzugt Cyclohexanol.

Das Gewichtsverhältnis von Wasser zu Alkohol liegt zwischen 2:1 und 0,5:1, vorzugsweise zwischen 1,5:1 und 0,8:1.

Die erfindungsgemäß einzusetzenden schwerlöslichen Alkohole werden zur besseren Aufarbeitung zugesetzt, da die Mischung Phenol/Alkohol eine geringere Dichte als die wäßrige Lösung hat und somit die organische über der wäßrigen Phase ist. Somit kann die wäßrige Phase unten abgelassen werden, die organische Phase, die das Phenolat enthält, kann dann im selben Trenngefäß mit demineralisiertem Wasser gewaschen werden und das Waschwasser wiederum unten abgelassen werden.

Setzt man den Alkohol nicht zu, dann ist nur die salzhaltige wäßrige Lösung schwerer als die organische Phase und kann unten abgelassen werden. Bei der weiteren Wäsche mit demineralisiertem Wasser kommt es zur Phasenumkehr, die organische Phase ist schwerer und daher unter der wäßrigen Phase. Diese Aufarbeitung erweist sich dahingehend als aufwendiger, daß ein 2. Aufarbeitungsgefäß notwendig ist.

Für die erfindungsgemäße Umsetzung eignen sich insbesondere Phosphoniumhalogenide der Formel (I) 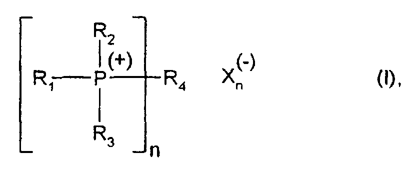 worin
- R₁ bis R₄: gleich oder verschieden, jeweils fiir einen C₁-C₁₂-Alkyl-, C₅-C₆-Cycloalkyl-, C₇-C₁₂-Aralkyl- oder C₆-C₁₄-Aryl-Rest steht,
worin
X⁽⁻⁾ fiir ein Halogenion, vorzugsweise F⁽⁻⁾, Cl⁽⁻⁾ oder Br⁽⁻⁾ steht, und n für 1 oder 2 steht, wobei für n = 2 R₄ für einen C₂-C₁₂-Alkylenrest steht.

Bevorzugt stehen die Reste R₁ bis R₄, gleich oder verschieden, jeweils für einen C₆-C₁₄-Aryl-Rest oder die Reste R₁ bis R₃ jeweils für einen C₆-C₁₄-Arylrest und R₄ fiir einen C₂-C₁₂-Alkylenrest.

Derartige Phosphoniumhalogenide und ihre Herstellung sind bekannt (siehe beispielsweise "Houben-Weyl, Methoden der organischen Chemie" Band XII/1, Seiten 79 ff. und Worrall, J. Amer. Chem. Soc. 52 (1930), Seiten 293 ff.).

Diese Verbindungen (I) entstehen bei der Umsetzung von Trialkyl- oder Triarylphosphinen, beispielsweise von Triphenylphosphin mit Halogenarylen oder Halogenalkylen, z.B. Benzylbromid in Gegenwart von Metallsalzen (Friedel-Crafts-Alkylierung) oder in Gegenwart von Grignard-Verbindungen und Kobalt(II)-chlorid.

Bevorzugte Phenole sind die der Formel (II) 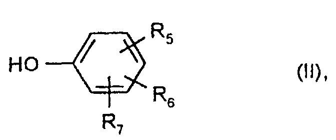 worin
- R₅ bis R₇: unabhängig voneinander H, C₁-C₁₂-Alkyl, C₅-C₆-Cycloalkyl, C₇-C₁₂-Arylalkyl und C₆-C₁₄-Aryl sind; vorzugsweise sind R₅ bis R₇ H.

Derartige Phenole sind literaturbekannt.

Bevorzugte Phosphoniumphenolate sind somit solche der Formel (III) 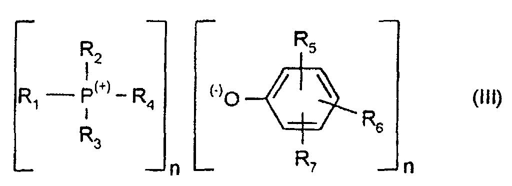 worin die Reste R₁ bis R₇ denen der Formeln (I) und (II) entsprechen und n wieder 1 oder 2 ist.

Für die Herstellung der wäßrig-alkalischen Phase wird bevorzugt demineralisiertes Wasser bzw. destilliertes Wasser verwendet.

Der pH-Wert von 9,5 bis 11,0, vorzugsweise von 9,5 bis 10,5, besonders bevorzugt von 10,0 bis 10,5 kann mit Hilfe einer Alkalilauge, vorzugsweise Natronlauge oder Kalilauge - unter Berücksichtigung der puffernden Wirkung von Phenol/Na-Phenolat - eingestellt werden.

Das erfindungsgemäße Verfahren kann kontinuierlich oder diskontinuierlich durchgeführt werden, wobei eine diskontinuierliche Arbeitsweise bevorzugt wird.

Nach einer bevorzugten Arbeitsweise wird Phenol, Phosphoniumhalogenid und Alkanol als Lösung vorgelegt und Wasser zugegeben. Gegebenenfalls unter Kühlung wird der pH-Wert unter Zugabe von Alkalilauge auf Werte von 9,5 bis 11,0, vorzugsweise von 9,5 bis 10,5, besonders bevorzugt von 10,0 bis 10,5 eingestellt. Dabei wird vorzugsweise unter intensiver Mischung der Reaktionskomponenten die Temperatur von 0 bis 55°C, vorzugsweise 15 bis 50°C gehalten. Die Reaktionsdauer sollte auf 2 Stunden, vorzugsweise auf 1 Stunde bemessen werden.

Das erfindungsgemäß hergestellte Phosphoniumphenolat wird vorzugsweise isoliert, indem man unter Verwendung des in Wasser schwerlöslichen Alkohols die wäßrige Phase von der organischen Phase trennt, letztere mindestens 1 x vorzugsweise 3 x mit demineralisiertem Wasser bzw. destilliertem Wasser extrahiert, anschließend den Alkohol beispielsweise destillativ entfernt und das Reaktionsprodukt nach Entfernung des Phenols trocknet. Sofern bei der erfindungsgemäßen Zweiphasengrenzflächenreaktion das Phosphoniumphenolat bereits in kristalliner Form anfällt, können diese Kristalle der üblichen Aufarbeitung, u.a. durch Auswaschen mit demineralisiertem Wasser oder destilliertem Wasser, gegebenenflals nach Umkristallisieren und Trocknen, gewonnen werden.

Wird kein schwerlöslicher Alkohol verwendet, werden die ausgefallenen Kristalle mit demineralisiertem bzw. destilliertem Wasser extrahiert.

Erfindungsgemäß hergestellte quartäre Phosphoniumphenolate sind insbesondere 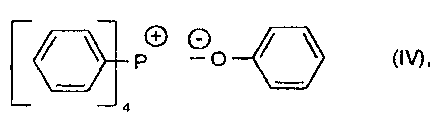 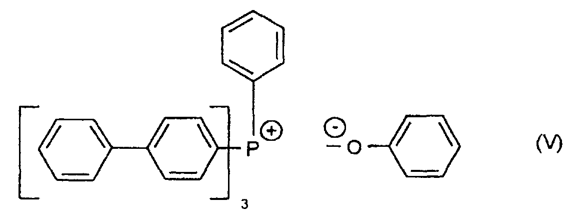 und

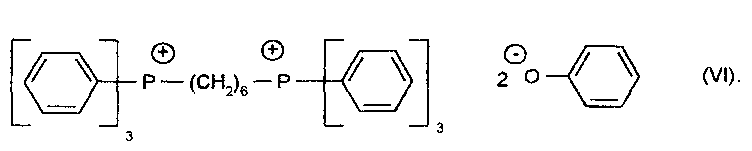

Die Verwendung der erfindungsgemäß erhältlichen Phosphoniumphenolate zur Herstellung von aromatischen Polycarbonaten kann in an sich bekannter Weise erfolgen (siehe US-A 3 442 854 loc. cit.).

Das Schmelzumesterungsverfahren geht bekanntlich beispielsweise aus von aromatischen Diphenolen, Kohlensäurediarylestern und gegebenenfalls Verzweigern und/oder Monophenolen.

Die erfindungsgemäß erhältlichen Phosphoniumphenolate werden hierbei als Katalysatoren in Mengen von 10⁻¹ mol bis 10⁻⁸ mol, vorzugsweise in Mengen von 10⁻³ mol bis 10⁻⁷ mol, pro mol Diphenol eingesetzt.

Weitere Einzelheiten des Schmelzumesterungsverfahrens sind in der Literatur beschrieben (siehe beispielsweise Hermann Schnell, Chemistry and Physics of Polycarbonates, Polymer Reviews, Vol. 9, 1964, Seiten 44 bis 51, DE-A 1 031 512, US-A 3 022 272, US-A 5 340 905 und US-A 5 399 659).

Die mit den erfindungsgemäß erhältlichen Phosphoniumphenolaten hergestellten thermoplastischen Polycarbonate sind lösungsmittelfrei, mit heller Eigenfarbe ausgestattet und weitgehend frei von unerwünschten Fehlstellen im Polycarbonat.

Der technische Einsatz dieser so hergestellten Polycarbonate in Form der verschiedensten Formteile ist überall dort möglich, wo bislang bereits thermoplastische Polycarbonate eingesetzt wurden, etwa in der Elektrotechnik, als Lampenabdeckungen, als Sicherheitsscheiben oder als CD-Material.

### Beispiele

### Vergleichsbeispiel (siehe Beispiel A von P 19635656.3 und von P 19723524.7)

In einem 2,5-1-Rundkolben mit Rührer, Thermometer, Rückflußkühler und Tropftrichter werden 88,0 g (1,0 mol) Phenol, 88,0 g (0,99 mol) 45%ige Natronlauge und 1,5 l 3E-Wasser vorgelegt und bei 20°C bis 25°C gerührt, der pH-Wert ist ca. 14. Zu dieser Lösung werden 41,93 g (0,10 mol) Tetraphenylphosphoniumbromid zugegeben. Anschließend wird mindestens 0,5 h gerührt. Nach der Phasentrennung wird die organische Phase 3 mal mit 3E-Wasser gewaschen und anschließend eingedampft. Der kristalline Rückstand wird bei 100°C im Vakuum getrocknet.

### Beispiel 1

In einem 2,5-1-Rundkolben mit Rührer, Thermometer und Tropftrichter werden 470 g (5,0 mol) Phenol, 1,0 l 3E-Wasser, 419,3 g (1 mol) Tetraphenylphosphoniumbromid und 800 g Isobutanol vorgelegt und bei 20°C bis 25°C gerührt. Innerhalb von 2 min. werden 106,7 g (1,2 mol) 45%ige Natronlauge zugetropft, der pH-Wert wird mit einer Glaselektrode kontrolliert, ere muß in einem Bereich von 9,5 bis 11,0 liegen. Anschließend wird mindestens 0,5 h bei 45°C gerührt. Nach der Phasentrennung wird die untere wäßrige Phase abgelassen und die organische Phase 3 mal mit 3E-Wasser gewaschen, das Waschwasser als schwerere Phase kann jeweils unten abgelassen werden. Anschließend wird das Isobutanol bei 50°C im Wasserstrahlvakuum abdestilliert.

Der kristalline Rückstand wird bei 100°C im Vakuum getrocknet. Die Ausbeute bestimmt nach der P-NMR Methode beträgt 98,2 % der theoretischen Ausbeute.

### Beispiel 2

In einem 5-1-Rundkolben mit Rührer, Thermometer und Tropftrichter werden in 940 g (10,0 mol) Phenol, 419,3 g (1 mol) Tetraphenylphosphoniumbromid bei 40 bis 45°C gelöst, anschließend werden 500 ml 3E-Wasser zugegeben. Innerhalb von 2 Minuten werden 133,3 g (1,5 mol) 45%ige Natronlauge zugetropft, der pH-Wert wird mit einer Glaselektrode kontrolliert, er muß in einem Bereich von 9,5 bis 11,0 liegen. Anschließend wird mindestens 0,5 h bei 40 bis 45°C gerührt. Nach der Phasentrennung wird die Wasserphase (untere Phase) abgetrennt und die organische Phase 3 mal mit 3E-Wasser gewaschen. Dabei kommt es zu einer Phasenumkehr, da die wäßrige Phase nun leichter als die organische Phase ist. Die organische Phase wird abgelassen und in einem weiteren Gefäß mit 3E-Wasser gewaschen, dieser Vorgang wird dreimal wiederholt. Man erhält eine phenolische Lösung mit 37% Tetraphenylphosphoniumphenolat.

Die Ausbeute bestimmt nach der P-NMR Methode beträgt 100 % der theoretischen Ausbeute.

### Verwendungsbeispiele

B1) In einem 500 ml Dreihalskolben mit Rührer, Innenthermometer und Vigreuxkolonne (30 cm, verspiegelt) mit Brücke werden 114,15 g (0,500 mol) Bisphenol A und 113,54 (0,530 mol) Diphenylcarbonat eingewogen. Die Apparatur wird durch Anlegen von Vakuum und Spülen mit Stickstoff (3 mal) vom Luftsauerstoff befreit und das Gemisch auf 150°C augeheizt. Jetzt werden 0,0173 g (4 x 10⁻³ mol-%) Tetraphenylphosphoniumphenolat (TPP-P) hergestellt gemäß Beispiel 1, bezogen auf Bisphenol A, als 3%ige phenolische Lösung zugegeben und das entstehende Phenol bei 100 mbar abdestilliert. Gleichzeitig wird die Temperatur bis auf 250°C gesteigert. Nun wird das Vakuum stufenweise bis auf 1 mbar verbessert und die Temperatur auf 260°C erhöht. Anschließend wird die Temperatur auf 280°C erhöht und bei 0,1 mbar 1,5 Stunden gerührt. Man erhält ein farbhelles lösungsmittelfreies Polycarbonat mit einer relativen Lösungsviskosität von 1,250 (Dichlormethan, 25°C, 5 g/l).
   Der Gehalt an Verzweiger der Formel (VII) im hergestellten Polycarbonat beträgt 25 ppm. Der phenolische OH-Wert des Polycarbonats beträgt 70 ppm. 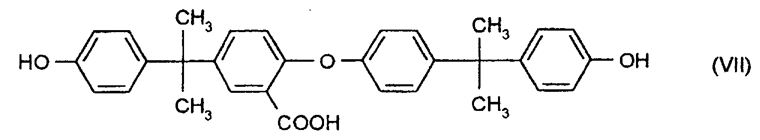
B2) Wie Beispiel B1), nur wird die Temperatur von 260°C auf 300°C erhöht und bei 0,1 mbar 1,5 Stunden gerührt. Man erhält ein farbhelles, lösungsmittelfreies Polycarbonat mit einer relativen Lösungsviskosität von 1,300 (Dichlormethan, 25°C, 5 g/l). Der Gehalt an Verzweiger der Formel (VII) im hergestellten Polycarbonat beträgt 18 ppm. Der phenolische OH-Wert des Polycarbonats beträgt 55 ppm.

## Patentansprüche

1. Verfahren zur Herstellung von Phosphoniumphenolaten aus Phosphoniumhalogeniden und Phenolen in wäßrig-alkalischer Lösung bei Temperaturen von 0 bis 55°C, **dadurch gekennzeichnet, daß** man die Umsetzung im Molverhältnis Phenol zu Phosphoniumhalogenid zwischen 2:1 und 10:1 bei pH-Werten von 9,5 bis 11 und in Gegenwart von Alkoholen in Mengen von 50 Gew.-% bis 200 Gew.-%, bezogen auf die Masse der wäßrigen Phase, durchführt, wobei die Alkohole eine Löslichkeit in reinem Wasser von maximal 15 Gew.-% haben.

2. Verfahren gemäß Anspruch 1, **dadurch gekennzeichnet, daß** man bei pH-Werten von 9,5 bis 10,5 arbeitet.

3. Verfahren gemäß Anspruch 1, **dadurch gekennzeichnet, daß** man bei pH-Werten von 10 bis 10,5 arbeitet.

4. Verfahren gemäß Anspruch 1, **dadurch gekennzeichnet, daß** man in Gegenwart von Alkoholen in Mengen von 66 Gew.-% bis 125 Gew.-% umsetzt.

## Claims

1. A process for preparing phosphonium phenolates from phosphonium halides and phenols in aqueous alkaline solution at temperatures of 0 to 55°C, **characterised in that** the reaction is performed with a molar ratio of phenol to phosphonium halidebetween 2:1 and 10:1 at a pH of 9.5 to 11 and in the presence of alcohols in amounts of 50 wt.% to 200 wt.%, with respect to the weight of the aqueous phase, wherein the alcohols have a solubility in pure water of at most 15 wt.%.

2. A process according to Claim 1, **characterised in that** it is performed at a pH of 9.5 to 10.5.

3. A process according to Claim 1, **characterised in that** it is performed at a pH of 10 to 10.5.

4. A process according to Claim 1, **characterised in that** the reaction is performed in the presence of alcohols in amounts of 66 wt.% to 125 wt.%.

## Revendications

1. Procédé pour la préparation de phénolates de phosphonium à partir d'halogénures de phosphonium et de phénols en solution aqueuse-alcaline à des températures de 0 à 55°C, **caractérisé en ce que** l'on réalise la réaction dans un rapport molaire phénol à halogénure de phosphonium compris entre 2:1 et 10:1 pour des valeurs de pH de 9,5 à 11 et en présence d'alcools dans des quantités de 50 % en poids à 200 % en poids, rapportés à la masse de la phase aqueuse, les alcools ayant une solubilité dans l'eau pure au maximum de 15 % en poids.

2. Procédé selon la revendication 1, **caractérisé en ce que** l'on travaille à des valeurs de pH de 9,5 à 10,5.

3. Procédé selon la revendication 1, **caractérisé en ce que** l'on travaille à des valeurs de pH de 10 à 10,5.

4. Procédé selon la revendication 1, **caractérisé en ce que** l'on réalise la réaction en présence d'alcools dans des quantités de 66 % en poids à 125 % en poids.
